# EUROPÄISCHE PATENTANMELDUNG

(11) **EP 2 896 406 A2**
(43) Veröffentlichungstag der Anmeldung: **22.07.2015**
(21) Anmeldenummer: 14196903.0
(22) Anmeldetag: 09.12.2014
(51) Int. Cl.: A61L 2/26, A61L 2/20, A61J 1/00, B65D 75/32

(54) **Verpackungsstruktur und Verfahren zur sterilen Verpackung von Behältern für Substanzen für medizinische, pharmazeutische oder kosmetische Anwendungen sowie Verfahren zur Weiterverarbeitung von Behältern unter Verwendung der Verpackungsstruktur**

(30) Priorität: 27.12.2013 DE 102013114896
(71) Anmelder: Schott AG, 55122 Mainz (DE)
(72) Erfinder: Deutschle, Gregor Fritz, 65185 Wiesbaden (DE); Pawlowski, Edgar, 55271 Stadecken-Elsheim (DE); Wassenberg, Jörn, 55130 Mainz (DE); Wissner, Kai, 69493 Hirschberg (DE); Reisse, Andreas, 93057 Regensburg (DE)
(74) Vertreter: 2K Patentanwälte Blasberg Kewitz & Reichel

(57) **Zusammenfassung**

Offenbart wird eine Verpackungsstruktur in der Behälter in einfacher Weise sterilisiert werden können und anschließend sicher und ohne Glaskontakt steril transportiert werden.

Ein Verfahren zur sterilen Verpackung einer Mehrzahl von Behältern (2) umfasst die folgenden Schritte: Bereitstellen eines Trägers (35), in welchem eine Mehrzahl von Aufnahmen (34) ausgebildet ist, wobei die Aufnahmen jeweils von einem geschlossenen Boden (33) und einer umlaufenden Seitenwand (32) ausgebildet sind, die dem jeweiligen Boden gegenüberliegenden oberen Enden der Aufnahmen offen ausgebildet sind und an den oberen Enden der Aufnahmen umlaufend ausgebildete Verbindungsstege (35) vorgesehen sind; Anordnen der Mehrzahl von Behältern (2) in den Aufnahmen (34) des Trägers; Bereitstellen einer gasundurchlässigen Schutzfolie (50; Verbinden der Schutzfolie entlang den Verbindungsstegen mit der Oberseite des Trägers (S5), um sämtliche Aufnahmen (34) mit den darin jeweils einzeln aufgenommenen Behältern (2) zu verpacken; und Sterilisieren der Aufnahmen (34) mit den darin aufgenommenen Behältern und/oder der Innenräume der Behälter durch Einströmen eines Gases (S4) in die Aufnahmen (34) des Trägers und/oder in die Innenräume der Behälter durch zumindest einen gasdurchlässigen Abschnitt (54; 540), der als Abschnitt der gasundurchlässigen Schutzfolie (50) oder der Verpackungsstruktur (30) ausgebildet ist.

Von der Verpackungsstruktur können Verpackungs-Untereinheiten mit jeweils nur einer Aufnahme abgetrennt werden, wobei die Behälter in den abgetrennten Verpackungs-Untereinheiten auch weiterhin steril gegen die Umgebung verpackt aufgenommen ist.

## Beschreibung

Die vorliegende Anmeldung beansprucht die Priorität der Deutschen Patentanmeldung 10 2013 114 896.4 "Verpackungsstruktur und Verfahren zur sterilen Verpackung von Behältern für Substanzen für medizinische, pharmazeutische oder kosmetische Anwendungen sowie Verfahren zur Weiterverarbeitung von Behältern unter Verwendung der Verpackungsstruktur", angemeldet am 27.12.2013, deren gesamter Inhalt hiermit im Wege der Bezugnahme mit beinhaltet sei.

### GEBIET DER ERFINDUNG

Die vorliegende Erfindung betrifft allgemein die sterile Verpackung einer Mehrzahl von Behältern zur Aufbewahrung von Substanzen für medizinische, pharmazeutische oder kosmetische Anwendungen, insbesondere von Fläschchen, Ampullen oder Karpulen, und betrifft insbesondere eine Verpackungsstruktur, in der Behälter in einfacher Weise sterilisiert werden können und anschließend sicher und ohne Glaskontakt steril transportiert werden können.

### HINTERGRUND DER ERFINDUNG

Die Fig. 14a der DE 10 2012 103 896 A1 der Anmelderin offenbart eine Verpackungsstruktur für Behälter für medizinische, pharmazeutische oder kosmetische Anwendungen, die in den Aufnahmen der Verpackungsstruktur angeordnet sind. Die Verpackungsstruktur ist durch Aufbringen Schutzfolie, die selektiv gasdurchlässig ist, gegen die Umgebung steril verpackt. Durch die Schutzfolie kann ein Gas einströmen, um die Aufnahmen und/oder Innenräume der Behälter durch Begasung zu sterilisieren. Um einzelne Behälter zu entnehmen, muss die Schutzfolie aufgebrochen werden. In diesem Zustand sind die Behälter nicht mehr steril aufbewahrt. Wenn nur ein Behälter oder wenige Behälter entnommen werden sollen, müssen die restlichen Behälter entweder entsorgt oder vor deren Weiterverarbeitung erneut sterilisiert und wieder verpackt werden, um strenge Hygienevorschriften einzuhalten.

US 200802511411 A1 offenbart eine Blisterverpackung für inhalationsfähige Medikamente, bei der die Medikamente unmittelbar in konkave Vertiefungen eines Substrats eingebracht werden, das mit einer Folie versiegelt wird. Die Verpackungen einzelner Medikamentendosen können einzeln aufgebrochen oder von der Blisterverpackung abgetrennt werden. Eine Aufnahme von Behältern für medizinische, pharmazeutische oder kosmetische Anwendungen in den konkaven Vertiefungen des Substrats ist jedoch nicht möglich.

WO 2011104385 A1 offenbart eine Sichtverpackung aus Pappe oder Karton mit einem Sichtfenster zur visuellen Inspektion der aufbewahrten Arzneimittel. Die Arzneimittel sind ihrerseits in sterilen Verpackungen verpackt. Die Aufnahme mehrerer Arzneimitteldosen in der Sichtverpackung ist nicht offenbart.

EP 2092927 B1 offenbart eine Verpackungseinheit mit mehreren Blisterverpackungen, die in einem Hochregallager automatisiert gelagert und vereinzelt ausgegeben werden können. Eine Aufnahme von Behältern für medizinische, pharmazeutische oder kosmetische Anwendungen in den Blisterverpackungen ist jedoch nicht möglich.

WO 2013110107 A1 offenbart eine Medikamentenverpackung zur Aufnahme mehrerer geblisterter Einheiten, die eine Antenne aufweist, um Informationen, welche die Einheiten betreffen, automatisch auszulesen. Eine Vereinzelung der geblisterten Einheiten sowie ein Sterilisieren des Innenraums der Medikamentenverpackung sind nicht offenbart.

Hinsichtlich der Verpackung und des sterilen Transports von Behältern für medizinische, pharmazeutische oder kosmetische Anwendungen besteht weiterer Verbesserungsbedarf.

### ZUSAMMENFASSUNG DER ERFINDUNG

Aufgabe der vorliegenden Erfindung ist es, eine verbesserte Verpackungsstruktur zur sterilen Verpackung einer Mehrzahl von Behältern für medizinische, pharmazeutische oder kosmetische Anwendungen bereitzustellen, die in einfacher Weise und kostengünstiger Weise hergestellt und flexibler eingesetzt werden kann. Gemäß weiteren Gesichtspunkten der vorliegenden Erfindung sollen ferner ein entsprechendes Verfahren zur sterilen Verpackung einer Mehrzahl von Behältern, ein Verfahren zur Bearbeitung oder Verarbeitung von Behältern mit einer solchen Verpackungsstruktur sowie die Verwendung einer Schutzfolie für eine solche Verpackungsstruktur bereitgestellt werden.

Diese Aufgaben werden gemäß der vorliegenden Erfindung durch eine Verpackungsstruktur mit den Merkmalen nach Anspruch 1, durch ein Verfahren zur sterilen Verpackung einer Mehrzahl von Behältern für medizinische, pharmazeutische oder kosmetische Anwendungen nach Anspruch 7, ein Verfahren zur Bearbeitung oder Verarbeitung von Behältern nach Anspruch 14, sowie die Verwendung einer Schutzfolie nach Anspruch 15, gelöst. Weitere vorteilhafte Ausführungsformen sind Gegenstand der rückbezogenen Unteransprüche.

Gemäß einem ersten Gesichtspunkt der vorliegenden Erfindung wird ein Verfahren zur sterilen Verpackung einer Mehrzahl von Behältern für medizinische, pharmazeutische oder kosmetische Anwendungen bereitgestellt, mit den folgenden Schritten: Bereitstellen eines Trägers, in welchem eine Mehrzahl von Aufnahmen ausgebildet ist, wobei die Aufnahmen jeweils von einem geschlossenen Boden und einer umlaufenden Seitenwand ausgebildet sind, die dem jeweiligen Boden gegenüberliegenden oberen Enden der Aufnahmen offen ausgebildet sind und an den oberen Enden der Aufnahmen umlaufend ausgebildete Verbindungsstege vorgesehen sind, die gemeinsam eine Oberseite des Trägers ausbilden; Anordnen der Mehrzahl von Behältern in den Aufnahmen des Trägers; Bereitstellen einer gasundurchlässigen Schutzfolie; Verbinden der Schutzfolie entlang den Verbindungsstegen mit der Oberseite des Trägers, um sämtliche Aufnahmen mit den darin jeweils einzeln aufgenommenen Behältern zu verpacken; und Sterilisieren der Aufnahmen mit den darin aufgenommenen Behältern und/oder der Innenräume der Behälter durch Einströmen eines Gases in die Aufnahmen des Trägers und/oder in die Innenräume der Behälter durch zumindest einen gasdurchlässigen Abschnitt, wobei der zumindest eine gasdurchlässige Abschnitt als Abschnitt der gasundurchlässigen Schutzfolie oder der Verpackungsstruktur ausgebildet ist.

Die Behälter können in der Verpackungsstruktur sicher und ohne Glaskontakt zueinander steril transportiert werden. Zwischen den einzelnen Verpackungs-Untereinheiten sind bevorzugt Schwächungsbereiche in der Verpackungsstruktur eingelassen oder ausgebildet, beispielsweise durch Prägen oder Laserbearbeitung des Materials der Verpackungsstruktur. Damit können einzelne Verpackungs-Untereinheiten mit jeweils nur einer Aufnahme von der Verpackungsstruktur in einfacher Weise abgetrennt werden, wobei die Behälter in den Aufnahmen der abgetrennten Verpackungs-Untereinheiten auch weiterhin steril gegen die Umgebung verpackt aufgenommen sind. Anders als nach dem Stand der Technik muss eine einmal geöffnete Verpackungsstruktur daher nicht mehr komplett verarbeitet oder genutzt werden. Die Aufnahmen können mit hoher Packungsdichte in einer regelmäßigen Anordnung an dem Träger angeordnet werden.

Gemäß einer bevorzugten Ausführungsform werden die Aufnahmen durch Einströmen eines Gases durch zumindest einen gasdurchlässigen Abschnitt, die in der Schutzfolie ausgebildet oder mit dieser in geeigneter Weise verbunden ist, sterilisiert oder entkeimt werden. Gemäß einer weiteren Ausführungsform können die selektiv gasdurchlässigen Abschnitte auch unmittelbar in den Seitenwänden oder den Böden der Aufnahmen der Verpackungsstruktur ausgebildet sein.

Gemäß einem weiteren Gesichtspunkt der vorliegenden Erfindung wird eine entsprechend ausgelegte Verpackungsstruktur bereitgestellt, wie nachstehend ausgeführt.

Gemäß einem weiteren Gesichtspunkt der vorliegenden Erfindung wird ein Verfahren zur sterilen Verpackung einer Mehrzahl von Behältern für medizinische, pharmazeutische oder kosmetische Anwendungen bereitgestellt, mit den folgenden Schritten: Bereitstellen eines Trägers, wie vorstehend ausgeführt; Anordnen der Mehrzahl von Behältern in den Aufnahmen des Trägers; Bereitstellen einer Schutzfolie, die zumindest einen gasundurchlässigen Abschnitt aufweist, um die Aufnahmen steril zu verpacken; Verbinden der Schutzfolie entlang den Verbindungsstegen mit der Oberseite des Trägers, um sämtliche Aufnahmen mit den darin jeweils einzeln aufgenommenen Behältern jeweils einzeln steril zu verpacken und eine Verpackungsstruktur wie vorstehend beschrieben auszubilden.

Gemäß einer bevorzugten weiteren Ausführungsform können die Aufnahmen nachträglich durch Einströmen eines Gases durch zumindest einen gasdurchlässigen Abschnitt in der vorstehend beschriebenen Weise sterilisiert bzw. entkeimt werden.

Gemäß einer weiteren Ausführungsform ist die Verpackungsstruktur bevorzugt aus einem Material hergestellt sein, welches Wasserstoffperoxid (H₂O₂) Dampf (VHP) resistent ist. Geeignet sind besondere chemikalienresistente Kunststoffe, wie beispielsweise Polyamid (PA), Polyethylen (PE), Polycarbonat (PC), Polypropylen (PP), PSU und PVC. Geeignet ist auch eine Metallfolie oder Aluminiumfolie. Besonders geeignet ist ein Aluminium-Polypropylen-Verbundmaterial als Folie. Wasserstoffperoxid (VHP) wirkt entkeimend, kann in vorteilhaft einfacher und kostengünstiger Weise durch aktives Verdampfen einer wässrigen Wasserstoffperoxid-Lösung erzeugt werden und so zum Sterilisieren der Innenräume der Verpackungsstruktur und/oder der Innenräume der darin aufzunehmenden Behälter eingesetzt werden. Um eine hohe biologische Dekontaminationsrate der Mikroorganismen zu erreichen, ist eine definierte hohe Wasserstoffperoxid-Konzentration von 30% bis 35% erforderlich.

Gemäß einem weiteren Gesichtspunkt der vorliegenden Erfindung wird ein Verfahren zur Bearbeitung oder Verarbeitung von Behältern für medizinische, pharmazeutische oder kosmetische Anwendungen unter Verwendung einer Verpackungsstruktur, wie vorstehend beschrieben, mit den folgenden Schritten: Bereitstellen der Verpackungsstruktur mit einer Mehrzahl von Aufnahmen, in denen jeweils ein Behälter steril verpackt aufgenommen ist; Abnehmen oder Öffnen der Schutzfolie von zumindest einer Aufnahme der Verpackungsstruktur; Bearbeitung oder Verarbeitung des jeweiligen Behälters der zumindest einen Aufnahme; und Aufbringen der zuvor abgenommenen oder geöffneten Schutzfolie oder einer anderen Schutzfolie, um die zumindest eine Aufnahme zu verschließen, bevorzugt steril zu verschließen.

Ein weiterer Gesichtspunkt der vorliegenden Erfindung betrifft die Verwendung einer Schutzfolie mit zumindest einem gasundurchlässigen Abschnitt und zumindest einem gasdurchlässigen Abschnitt, wie vorstehend beschrieben, zur Herstellung einer solchen Verpackungsstruktur.

### FIGURENÜBERSICHT

Nachfolgend wird die Erfindung in beispielhafter Weise und unter Bezugnahme auf die beigefügten Zeichnungen beschrieben werden, woraus sich weitere Merkmale, Vorteile und zu lösende Aufgaben ergeben werden. Es zeigen:
- Fig.1: eine Verpackungsstruktur gemäß einer ersten Ausführungsform der vorliegenden Erfindung;
- Fig. 2: eine Verpackungsstruktur gemäß einer weiteren Ausführungsform der vorliegenden Erfindung;
- Fig. 3a: in einer schematischen Schnittansicht eine Verpackungsstruktur gemäß einer weiteren Ausführungsform der vorliegenden Erfindung;
- Fig. 3b: in einer schematischen Schnittansicht Strömungspfade, entlang denen ein Gas beim Sterilisieren der Verpackungsstruktur gemäß der Fig. 3a durch Begasen strömt;
- Fig. 4a - 4b: in einer schematischen Draufsicht und einem Teilschnitt eine Verpackungsstruktur gemäß einer weiteren Ausführungsform der vorliegenden Erfindung;
- Fig. 4c: in einem schematischen Teilschnitt eine Verpackungsstruktur gemäß einer weiteren Ausführungsform der vorliegenden Erfindung;
- Fig. 5a - 5f: in schematischen Teilschnitten weitere Einzelheiten einer Verpackungsstruktur gemäß weiteren Ausführungsformen der vorliegenden Erfindung;
- Fig. 6a - 6b: in einer Draufsicht eine Schutzfolie gemäß weiteren Ausführungsformen der vorliegenden Erfindung zur Verwendung bei einer Verpackungsstruktur;
- Fig. 6c - 6d: weitere Einzelheiten einer Schutzfolie gemäß weiteren Ausführungsformen der vorliegenden Erfindung;
- Fig. 7: ein schematisches Flussdiagramm eines Verfahrens zur sterilen Verpackung einer Mehrzahl von Behältern gemäß der vorliegenden Erfindung; und
- Fig. 8: in einem perspektivischen Teilschnitt und in Draufsicht einen Transport- und Verpackungsbehälter, in welchem eine Verpackungsstruktur gemäß einer weiteren Ausführungsform der vorliegenden Erfindung aufgenommen ist.

In den Figuren bezeichnen identische Bezugszeichen identische oder im Wesentlichen gleichwirkende Elemente oder Elementgruppen.

### Ausführliche Beschreibung von bevorzugten Ausführungsbeispielen

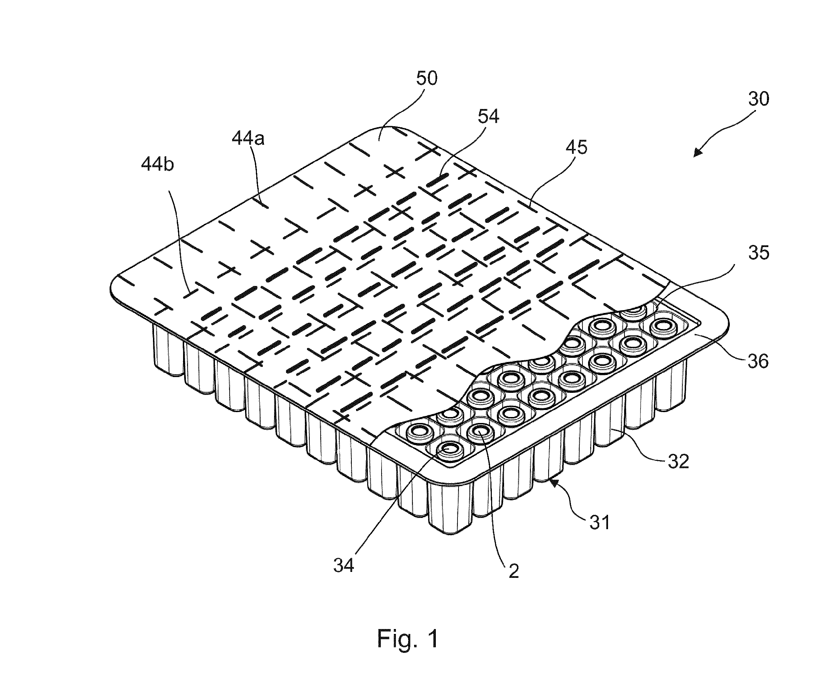

Die Fig. 1 zeigt in einer perspektivischen Draufsicht eine Verpackungsstruktur gemäß einer ersten Ausführungsform der vorliegenden Erfindung. Die Verpackungsstruktur 30 ist von einem Träger 31 und einer Schutzfolie 50 ausgebildet, die auf die Oberseite des Trägers aufgebracht ist, insbesondere aufgeklebt, aufgesiegelt, heißverklebt oder heißversiegelt ist oder auf diese durch Wärmeeinwirkung, insbesondere mittels Laser- oder rf-Strahlung, aufgeschmolzen ist. Der Träger 31 ist bevorzugt aus einem Kunststoff ausgebildet, insbesondere mittels eines Spritzgussverfahrens. Dieser Kunststoff kann flexibel oder starr sein. Der Träger 31 kann auch aus Verbundwerkstoffen ausgebildet sein, beispielsweise aus zellulosehaltigem Material, in das ein Kunststoffschlauch eingearbeitet ist, der die Innenwände der Aufnahmen ausbildet.

In dem Träger 31 ist eine Mehrzahl von Aufnahmen 34 ausgebildet, die jeweils insgesamt röhrenartig ausgebildet sind und einen geschlossenen Boden 33 und ein offenes oberes Ende an der Oberseite des Trägers 31 aufweisen. Genauer gesagt werden die Aufnahmen jeweils von dem geschlossenen Boden 33 und einer umlaufenden Seitenwand 32 ausgebildet. An den oberen Enden der Aufnahmen 34 sind umlaufend ausgebildete Verbindungsstege 35 vorgesehen, die gemeinsam die Oberseite des Trägers 31 ausbilden und über welche benachbarte Aufnahmen 34 miteinander verbunden sind. Die Verbindungsstege 35 sind bevorzugt flach ausgebildet und weisen eine Materialstärke auf, welche eine geeignete mechanische Stabilität oder auch Flexibilität der Verbindungsstege 35 vorgibt. Die Verbindungsstege 35 geben eine regelmäßige Anordnung der Aufnahmen 34 vor. Diese können insbesondere in einer regelmäßigen Anordnung von Reihen und Spalten von Aufnahmen angeordnet sein.

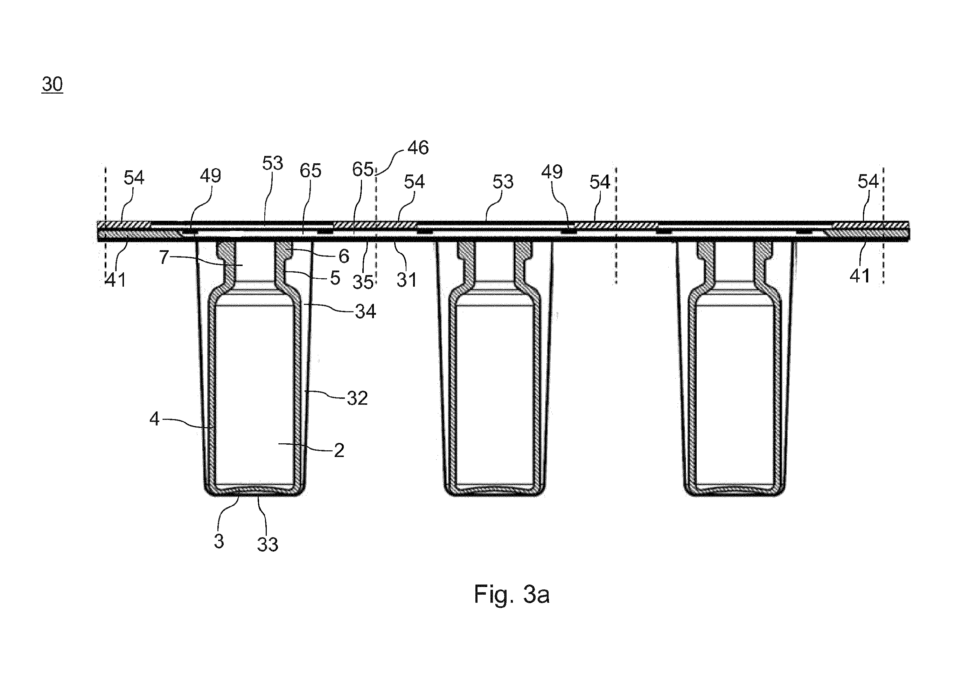

Die Schutzfolie 50 weist gasundurchlässige Abschnitte 53 auf (vgl. Fig. 3a), die jeweils oberhalb der Aufnahmen 34 ausgebildet sind und sich über die gesamte zugeordnete Aufnahme 34 sowie den zugeordneten umlaufenden Verbindungssteg 35 erstrecken, sodass durch Verbindung der Schutzfolie 50 mit den Verbindungsstegen 35 jeweils steril verpackte Verpackungs-Untereinheiten mit jeweils einer Aufnahme 34 ausgebildet werden können, die einzeln abgetrennt werden können und in denen jeweils ein einzelner Behälter 2 steril verpackt gegen die Umgebung aufgenommen ist. Hierzu können sich entlang den Verbindungsstegen 35 Schwächungsbereiche 44a, 44b erstrecken, entlang denen die Verbindungsstege 35 mechanisch abgetrennt, insbesondere abgebrochen, werden können. Solche Schwächungsbereiche 44a, 44b können entlang den Verbindungsstegen 35 beispielsweise als durchgehende, linienförmige Aussparungen oder als Reihe von punktförmigen Aussparungen ausgebildet sein, beispielsweise durch mechanisches Einritzen, Prägen oder durch Laserbearbeitung des Materials der Verbindungsstege 35. Derartige Schwächungsbereiche sind jedoch nicht zwingend erforderlich, da die Verpackungs-Untereinheiten bei einer Weiterverarbeitung der Verpackungsstruktur auch in anderer Weise abgetrennt werden können, beispielsweise durch mechanisches Abtrennen oder Lasertrennen.

In den Aufnahmen 34 sind jeweils einzelne Behälter 2 angeordnet. Die Schutzfolie 50 wird so mit der Oberseite des Trägers 31 entlang den Verbindungsstegen 35 und des Rands des Trägers 31 verbunden, dass die Behälter 2 in den von den Aufnahmen 34 ausgebildeten Verpackungs-Untereinheiten nach Verbindung der Schutzfolie 50 mit dem Träger 31 steril verpackt sind und dass die Verpackungs-Untereinheiten so von dem Träger 31 abgetrennt werden können, dass die einzelnen Behälter 2 auch in den abgetrennten Verpackungs-Untereinheiten weiterhin steril verpackt gegen die Umgebung aufgenommen sind. Hierzu ist auf eine ausreichende Breite der Verbindungsbereiche zu achten. Es muss sichergestellt sein, dass der Trennvorgang zum Abtrennen der Verpackungs-Untereinheiten präzise entlang dieser Verbindungsbereiche erfolgt, insbesondere mittig oder im Wesentlichen entlang dieser Verbindungsbereiche. Nach dem Abtrennen der Verpackungs-Untereinheiten muss die Breite des Verbindungsbereichs einer jeweiligen Aufnahme ausreichend sein, um eine sterile Verpackung des in der Aufnahme aufgenommenen Behälters zu gewährleisten.

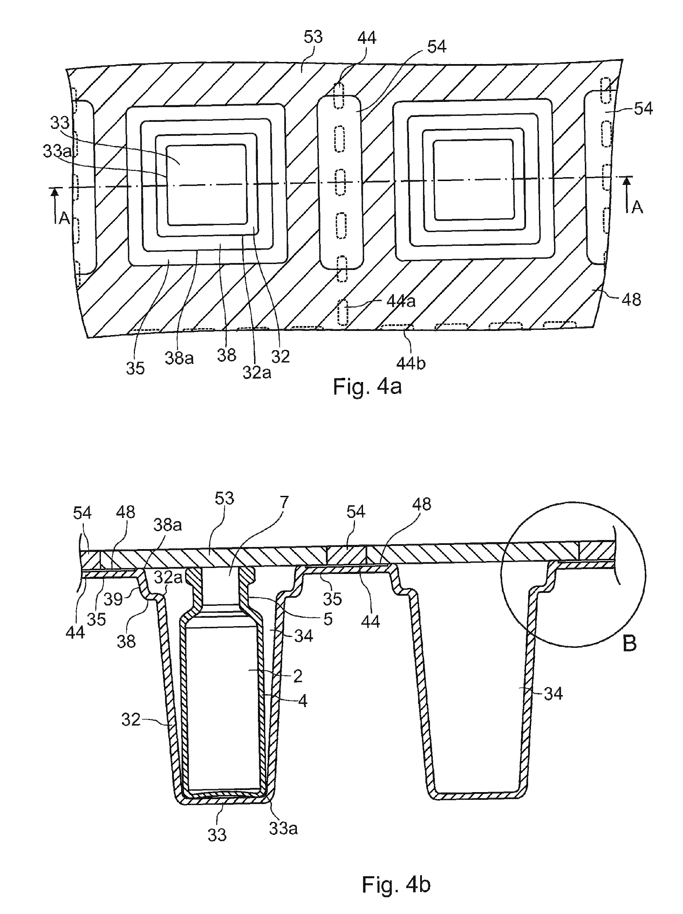

Wenn die Verpackungs-Untereinheiten mechanisch, insbesondere manuell, abgebrochen werden sollen, ist hierzu die Ausbildung der vorgenannten Schwächungsbereiche präzise entlang der Verbindungsstege 35 von Vorteil. Bevorzugt verlaufen die Schwächungsbereiche exakt mittig entlang der Verbindungsstege 35, sodass durch Vorgabe der Breite der Verbindungsstege 35 und der Überlappung des jeweiligen Verbindungsbereichs mit den Verbindungsstegen 35 eine ausreichende sterile Verpackung der Aufnahmen auch nach Abtrennen der Verpackungs-Untereinheiten gewährleistet werden kann, wie nachfolgend anhand der Fig. 4a ausführlicher beschrieben.

Ein Beispiel für Behälter in Gestalt von Fläschchen (englisch: vials) ist in einer Schnittansicht gemäß der Fig. 3a schematisch dargestellt. Die Fläschchen 2 weisen eine zylindrische Grundform auf, mit einer zylinderförmigen Seitenwand 4 mit - im Rahmen der Toleranzen - konstantem Innen- und Außendurchmesser, die von einem flach ausgebildeten Flaschenboden 3 senkrecht abragt und nahe dem oberen offenen Ende des Fläschchens in einen verengten Halsabschnitt 5 von vergleichsweise geringer axialer Länge und anschließend in einen verbreiterten oberen Rand 6 übergeht, der einen größeren Außendurchmesser aufweist als der zugeordnete Halsabschnitt 5 und zur Verbindung mit einem Verschlusselement ausgelegt ist. Derartige Fläschchen sind radial symmetrisch und aus einem durchsichtigen oder eingefärbten Glas oder auch durch Blasformen oder Kunststoff-Spritzgusstechniken aus einem geeigneten Kunststoffmaterial ausgebildet, und können grundsätzlich innenbeschichtet sein, so dass das Material des Fläschchens möglichst wenig Verunreinigungen an die aufzunehmende Substanz abgibt.

Ein weiteres Beispiel für Medikamentenbehälter im Sinne der vorliegenden Anmeldung sind Ampullen, Karpulen oder Spritzen- oder Injektionsbehältnisse.

Im Sinne der vorliegenden Erfindung dienen derartige Behälter (container) zur Aufbewahrung von Substanzen oder Wirkstoffen für medizinische, pharmazeutische oder kosmetische Anwendungen, die in einer oder auch mehreren Komponenten in fester oder flüssiger Form in dem Behälter aufbewahrt werden sollen. Gerade bei Glasbehältern können Aufbewahrungsdauern viele Jahre betragen, was insbesondere von der hydrolytischen Resistenz der verwendeten Glassorte abhängt. Während nachfolgend Behälter offenbart werden, die zylindrisch sind, sei darauf hingewiesen, dass die Behälter im Sinne der vorliegenden Erfindung auch ein anderes Profil haben können, beispielsweise ein quadratisches, rechteckförmiges oder vieleckiges Profil.

Wie man der Fig. 3a entnehmen kann, können die Seitenwände 32 der Aufnahmen zum unteren Ende hin konisch oder in anderer Weise aufeinander zulaufen und am unteren Ende einen Innendurchmesser aufweisen, der praktisch dem Außendurchmesser der aufzunehmenden Behälter 2 entspricht, sodass die Behälter 2 leicht in die Aufnahmen 34 hinein gleiten können und auf dem Boden 33 automatisch zentriert angeordnet sind. Wie in der Fig. 4b gezeigt, kann am oberen Ende der Aufnahmen 34 auch eine Stufe 38 ausgebildet sein, an der die Aufnahme aufgeweitet ist und von einer umlaufenden oberen Seitenwand 39 mit einem größeren Durchmesser begrenzt ist. Auf diese Weise können die Behälter zur Entnahme leichter an ihrem oberen Ende gegriffen werden.

Wie in der Fig. 1 gezeigt, weist die Schutzfolie 50 eine Mehrzahl von streifenförmigen gasdurchlässigen Abschnitten 54 auf, die jeweils einer Aufnahme 34 des Trägers 31 zugeordnet sind. Bei der in der Fig. 1 dargestellten Matrixanordnung der Aufnahmen 34 sind die gasdurchlässigen Abschnitte 54 parallel zu den Querseiten des Trägers 31 verlaufend angeordnet, wobei sich die gasdurchlässigen Abschnitte 54 nicht über die gesamte Länge der Querseite des Trägers 31 erstrecken. Die gasdurchlässigen Abschnitte 54 sind gemäß einer ersten Ausführungsform aus einer selektiv gasdurchlässigen Kunststofffolie ausgebildet, insbesondere einem Geflecht aus Kunststofffasern, beispielsweise aus Polypropylen-Fasern (PP) oder einer Tyvek®-Schutzfolie. Gemäß einer alternativen Ausführungsform sind die selektiv gasdurchlässigen Abschnitte 54 als gitterartige Strukturen mit Durchbrechungen ausgebildet, beispielsweise als Gitter, die von Kunststoffstegen ausgebildet sind, die sich mit einer vorbestimmten Gitterweite kreuzen. Nach dem Sterilisieren der Aufnahmen und/oder Behälter werden solche gitterartige Strukturen dann mittels eines gasundurchlässigen Abschnitts verschlossen, beispielsweise durch Aufkleben einer gasundurchlässigen Folie auf die jeweiligen Abschnitte mit den gitterartigen Strukturen. Die den Aufnahmen 34 zugeordneten gasundurchlässigen Abschnitte der Schutzfolie 50 sind beispielsweise aus einer Kunststoff- oder Metallfolie oder einem Verbundwerkstoff daraus ausgebildet. Die gasdurchlässigen Abschnitte 54 und die gasundurchlässigen Abschnitte können als Verbundmaterial auch einstückig ausgebildet sein.

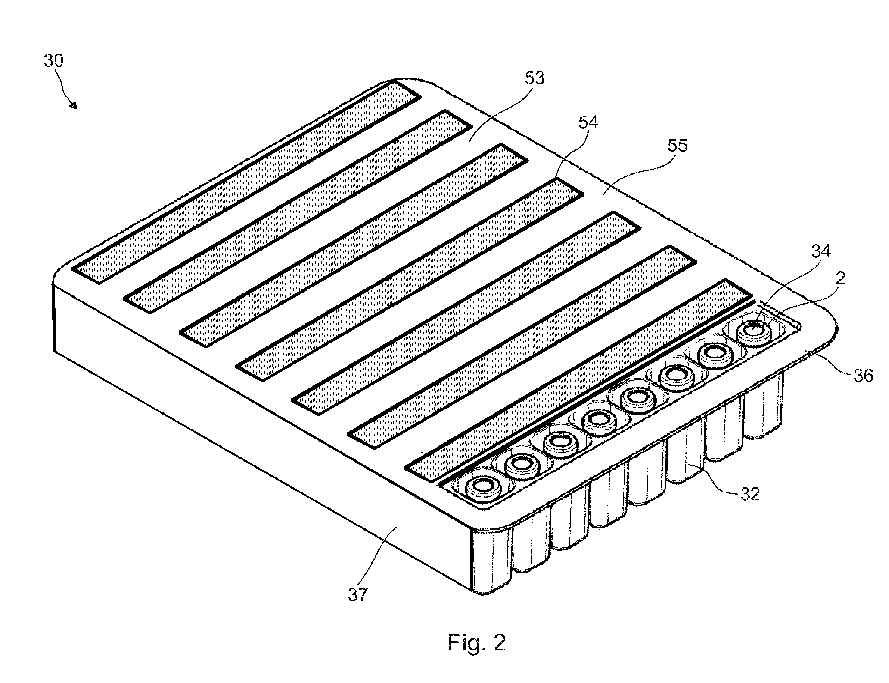

Die Fig. 2 zeigt eine weitere Ausführungsform einer Verpackungsstruktur 30, bei der die gasdurchlässigen Abschnitte 54 als Streifen ausgebildet sind, die sich entlang der gesamten Querseite des Trägers 31 und parallel und beabstandet zu den Spalten von Aufnahmen 34 des Trägers 31 erstrecken. Zwischen den Streifen von gasdurchlässigen Abschnitten 54 und den Aufnahmen sind jeweils Verbindungsstege 35 ausgebildet, die die Oberseite des Trägers 31 ausbilden, umlaufend ausgebildet sind und die Aufnahmen 34 umgeben. Die gasundurchlässigen Abschnitte 53 bedecken die Aufnahmen 34 vollständig und bilden auch die Ränder der Schutzfolie 50 aus. Abweichend zur Fig. 1 sind bei der Ausführungsform nach der Fig. 2 weitere Abdeckungen 37 entlang den Längsseiten (optional auch entlang den Querseiten) des Trägers 31 verlaufend vorgesehen, die einem weiteren mechanischen Schutz des Trägers 31 dienen.

Die Fig. 4a zeigt in einer vergrößerten Draufsicht zwei Aufnahmen 34. Dargestellt sind insbesondere auch die Kontur 33a des Bodens 33, die Kontur 32a der zylindrischen Seitenwand 32 der Aufnahmen 34 und die Kontur 38a der Stufe 38. Gemäß der Fig. 4a ist jede Aufnahme 34 von einem umlaufend ausgebildeten Verbindungssteg 35 umgeben, über den die Aufnahmen miteinander verbunden sind. Die Aufnahmen können von einem Bereich umgeben sein, in dem die Schutzfolie 50 nicht mit den Verbindungsstegen 35 verbunden ist. In den übrigen Abschnitten 48 der Verbindungsstege 35 ist jedoch die Schutzfolie 50 vollflächig mit den Verbindungsstegen 35 verbunden. Die Verbindung ist so gewählt, dass die Aufnahmen 34 des Trägers 31 jeweils gasdicht und steril gegen die gasdurchlässigen Abschnitte 54 sowie den Rand des Trägers abgedichtet sind. Hierzu können beispielsweise die folgenden Verbindungstechniken eingesetzt werden: Heißverklebung oder Heißversiegelung mittels eines Klebstoffs zwischen der Schutzfolie 50 und den Verbindungsstegen; abschnittsweises Verschmelzen der Schutzfolie 50 mit den Verbindungsstegen 35 oder einem darauf aufgebrachten Klebe- oder Verbindungsmittel, beispielsweise durch Einwirkung von Hitze und Verschmelzen von Kunststoffen. Die Verbindungsbereiche 48 umgeben die Aufnahmen 34 vollständig. Die Breite der Verbindungsbereiche 48 ist ausreichend gewählt, sodass die Aufnahmen 34 auch nach Abtrennung von einzelnen Verpackungs-Untereinheiten mit einzelnen Aufnahmen 34 noch zuverlässig gasdicht und steril gegen die Umgebung abgedichtet sind.

Bei dem Beispiel gemäß der Fig. 4a erfolgt die Abtrennung einzelner Verpackungs-Untereinheiten mit jeweils einer Aufnahme 34 durch mechanisches Abbrechen derselben entlang der horizontalen Schwächungslinien 44a und vertikalen Schwächungslinien 44b, die beispielsweise durch Ausbilden von Aussparungen durch mechanisches Prägen oder durch Laserbearbeitung entlang den Verbindungsstegen 35 ausgebildet werden. Die horizontalen Schwächungslinien 44a und vertikalen Schwächungslinien 44b sind dabei entsprechend der Matrixanordnung der Aufnahmen 34 ausgebildet und verlaufen bevorzugt exakt entlang der Mittellinie der jeweiligen Verbindungsstege 35. Die Schnittansicht gemäß der Fig. 4b zeigt Schwächungsbereiche 44 in Gestalt von konkaven Aussparungen, die in der Unterseite der Verbindungsstege 35 ausgebildet sind.

Bei dem Beispiel gemäß der Fig. 4a ist der selektiv gasdurchlässige Streifen 54 exakt symmetrisch bezüglich der horizontalen Schwächungslinie ausgebildet und kommuniziert mit der linken und rechten Aufnahme 34. Beim Abtrennen von Verpackungs-Untereinheiten werden diese gasdurchlässigen Abschnitte 54 mit durchtrennt, zu welchem Zweck auch in den gasdurchlässigen Abschnitten 54 vergleichbare Schwächungsbereiche (nicht dargestellt) ausgebildet sein können.

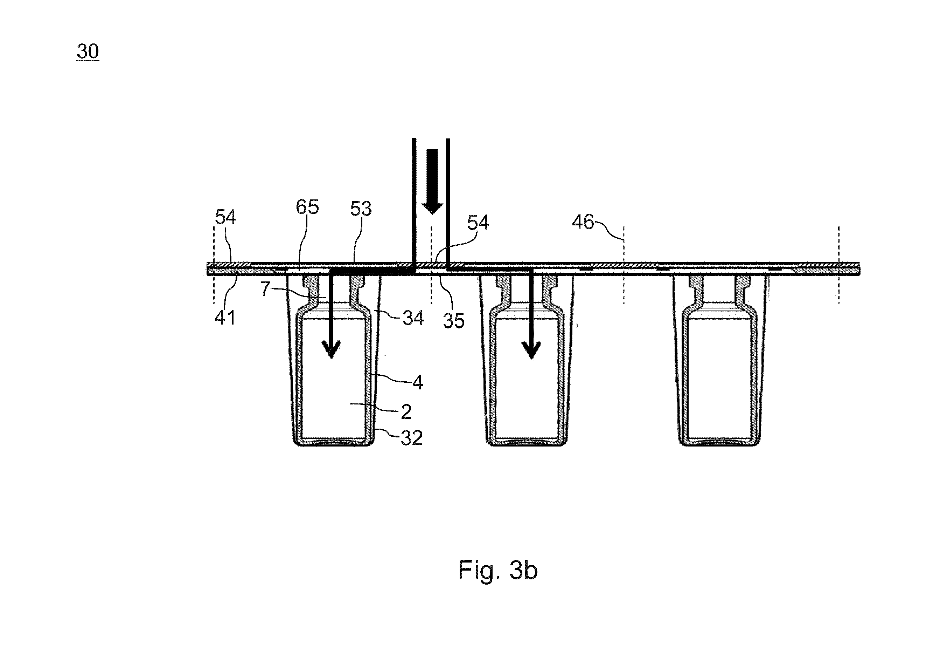

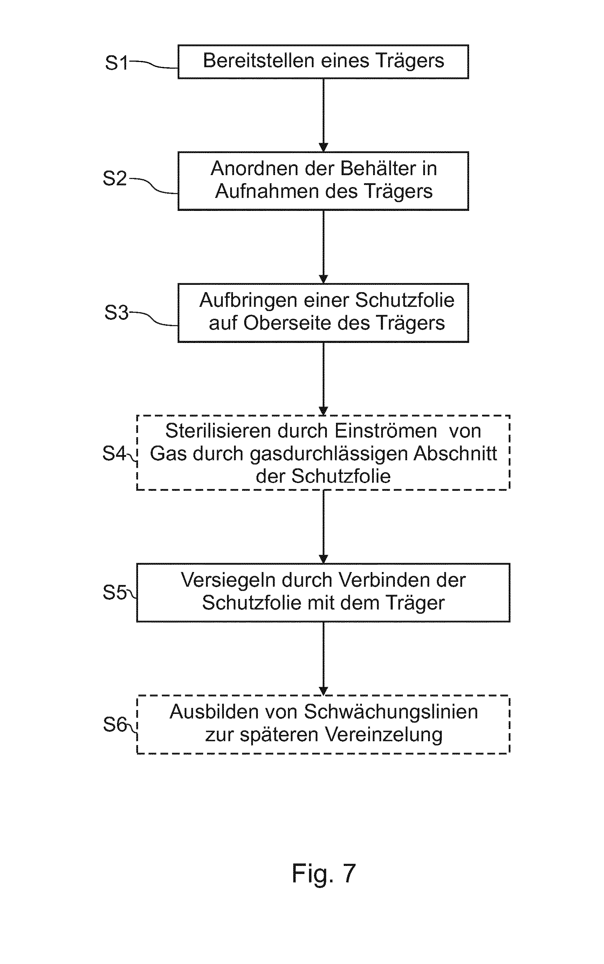

Anhand des Flussdiagramms gemäß der Fig. 7 sowie der Schnittansichten gemäß den Figuren 3a und 3b wird nachfolgend ein Verfahren gemäß der vorliegenden Erfindung zur sterilen Verpackung einer Mehrzahl von Behältern beschrieben.

Zunächst wird in dem Schritt S1 ein Träger 31 bereitgestellt, wie vorstehend anhand der Fig. 1 beschrieben. In die Aufnahmen 34 des Trägers 31 werden die Behälter 2 aufgenommen. Die Behälter können bereits befüllt und verschlossen sein.

Anschließend wird in dem Schritt S3 eine Schutzfolie 50 mit einer Mehrzahl von gasundurchlässigen Abschnitten 53 bereitgestellt, um die Aufnahmen steril zu verpacken. Die Schutzfolie 50 wird so in Bezug zu dem Träger 31 angeordnet, dass die gasundurchlässigen Abschnitte 53 exakt oberhalb der Aufnahmen angeordnet sind, um diese vollständig zu bedecken. Anschließend erfolgt in dem Schritt S5 ein Verbinden der Schutzfolie 50 entlang den Verbindungsstegen 35 mit der Oberseite des Trägers 31, um sämtliche Aufnahmen 34 mit den darin jeweils einzeln aufgenommenen Behältern 2 jeweils einzeln steril zu verpacken und eine Verpackungsstruktur 30 auszubilden, wie in den Figuren 1 oder 2 gezeigt. In dem Zustand gemäß den Figuren 1 und 2 können die Behälter steril verpackt und mechanisch geschützt gegen die Umgebung transportiert werden.

Bei einem bevorzugten Ausführungsbeispiel des Verfahrens gemäß der vorliegenden Erfindung sollen mittels der Verpackungsstruktur 30 noch offene und nicht befüllte Behälter 2 steril verpackt und transportiert werden. Zu diesem Zweck können die Aufnahmen 34 des Trägers 31 nach Einbringen der Behälter in dem Schritt S2 und Anordnen der Schutzfolie 50 auf der Oberseite des Trägers 31 in dem Schritt S3 durch Einströmen eines Gases in dem Schritt S4 sterilisiert werden, wie nachfolgend beschrieben.

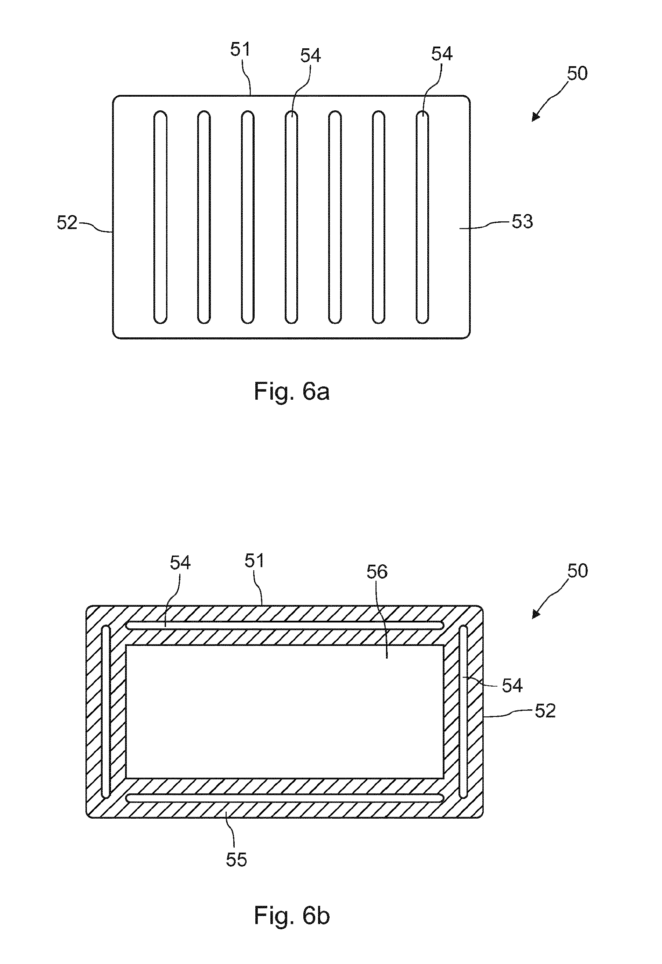

Zum Begasen der Innenräume der Aufnahmen 34 strömt ein Gas durch die gasdurchlässigen Abschnitte 54 der Schutzfolie 50 ein. Wie in der Fig. 3a gezeigt, kommunizieren die Aufnahmen 34 des Trägers 31 mit dem jeweils zugeordneten gasdurchlässigen Abschnitt 54 über Strömungskanäle 65, die zwischen der Oberseite des Trägers 31 und der Unterseite der Schutzfolie 50 verlaufen. Hierzu sind auf der Oberseite des Trägers 31 Abstandselemente 41 angeordnet, die bewirken, dass beim Auflegen der Schutzfolie 50 auf die Oberseite des Trägers 31 in dem Schritt S3 ein Spalt verbleibt. In diesem Zustand kann entlang den Längs- und Querseiten des Trägers 31 bereits ein umlaufender Verbindungsbereich ausgebildet werden, in dem die Schutzfolie 50 mit den Rändern des Trägers 31 verbunden ist, beispielsweise mittels Heißverklebung, Heißversiegelung oder thermischen Verschmelzens der Materialien von Schutzfolie 50 und Träger 31. In diesem Zustand wird von dem Verbindungsbereich ein umlaufender Rahmen ausgebildet, der beispielhaft in der Fig. 6b dargestellt ist und sich entlang den Längsseiten 51 und Querseiten 52 der Schutzfolie 50 umlaufend erstreckt. Dieser Rahmen schließt die gasdurchlässigen Abschnitte 54 vollständig ein, sodass das Gas, das durch diese in den vorgenannten Spalt 65 zwischen der Schutzfolie 50 und der Oberseite des Trägers 31 einströmt, nicht nach außen entweichen kann, sondern über die Strömungskanäle 65 in sämtliche zugeordnete Aufnahmen 34 des Trägers 31 strömt, um den Innenraum der Aufnahmen 34 und/oder der Behälter 2 zu sterilisieren.

Zum Sterilisieren kann beispielsweise Ethylenoxid (ETO) bei niedrigen Temperaturen (30°C-60°C) oder Wasserstoffperoxid (H₂O₂) Dampf (VHP) eingesetzt werden. Letzterer wirkt entkeimend und kann durch aktives Verdampfen einer wässrigen Wasserstoffperoxid-Lösung erzeugt werden. Um eine hohe biologische Dekontaminationsrate der Mikroorganismen zu erreichen, wird bevorzugt eine definierte hohe Konzentration von 30% bis 35% Wasserstoffperoxid eingesetzt.

Die Pfeile in der Fig. 3b stellen den Strömungsverlauf des einströmenden Gases dar.

Wie dem Fachmann ohne weiteres ersichtlich sein wird, können die vorgenannten Strömungskanäle auch in der Oberseite des Trägers 31 und/oder der Unterseite der Schutzfolie 50 in Gestalt von geeignet ausgebildeten Nuten oder Aussparung ausgebildet sein.

Nach dem Sterilisieren der Aufnahmen 34 in dem Schritt S4 erfolgt die Verbindung von Schutzfolie 50 und Oberseite des Trägers 31 in der vorstehend beschriebenen Weise entlang den Verbindungsstegen 35, um die Aufnahmen jeweils einzeln steril und gasdicht gegen den jeweils zugeordneten gasdurchlässigen Abschnitt zu verschließen.

Anschließend können in dem Schritt S6 Schwächungsbereiche entlang den Verbindungsstegen 35 ausgebildet werden, wie beispielhaft anhand der Fig. 1 vorstehend beschrieben.

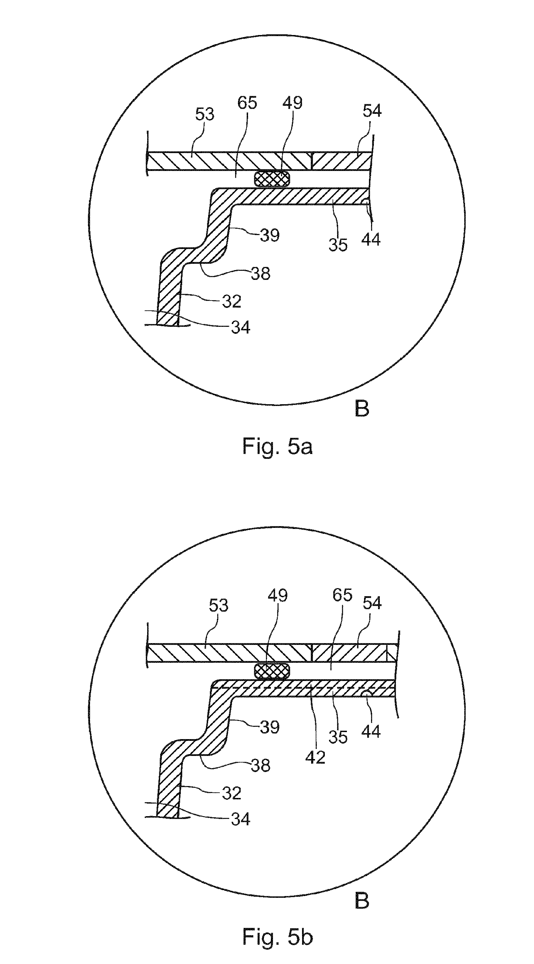

Nachfolgend werden anhand der Figuren 5a bis 5f weitere Einzelheiten bei der Ausbildung einer Verpackungsstruktur gemäß weiteren Ausführungsformen der vorliegenden Erfindung beschrieben. Dargestellt ist dabei jeweils in einem stark vergrößerten Teilschnitt der Bereich B gemäß der Fig. 4b.

Gemäß der Fig. 5a ist der Klebemittelstreifen 49 im Bereich des Strömungskanals 65, der die Aufnahme 34 mit dem zugeordneten gasdurchlässigen Abschnitt 54 verbindet, unterbrochen, so dass Gas durch diesen Bereich ungehindert in die Aufnahme 34 strömen kann. Der Klebemittelstreifen 49 kann auch durch Aufbringen einzelner Klebemittelpunkte unter regelmäßigen Abständen zueinander, entlang den Verbindungsstegen, ausgebildet werden. Zum Verkleben bzw. Verbinden ist dann dafür Sorge zu tragen, dass durch ausreichende Größe der Klebemitteltropfen und ausreichende Druck- und/oder Wärmeeinwirkung ein umlaufender und ausreichend breiter und dicker Verbindungsbereich 48 ausgebildet wird (vgl. Fig. 4a), der die jeweilige Aufnahme 34 vollständig gegen den zugeordneten gasdurchlässigen Abschnitt 54 und die Umgebung hin abdichtet. Wie man der Fig. 5a entnehmen kann, dient der Klebemittelstreifen 49 gleichzeitig als Abstandselement, um die Höhe des Strömungskanals 65 geeignet vorzugeben. Grundsätzlich kann so auch auf die in den Figuren 3a und 3b dargestellten seitlichen Abstandselemente 41 ganz verzichtet werden.

Gemäß der Fig. 5a ist der gasdurchlässige Abschnitt 54 außerhalb der Kontur des Klebemittelstreifens 49 angeordnet, während sich der gasundurchlässige Abschnitt 53 bis über die Kontur des Klebemittelstreifens 49 hinaus erstreckt. Nach Verbinden von Schutzfolie 50 und Träger 31 wird somit die Aufnahme 34 vollständig von dem Verbindungsbereich umgeben. Gemäß der Fig. 5a ist der Schwächungsbereich in Gestalt einer konkaven Aussparung 44 mittig unterhalb des gasdurchlässigen Abschnitts 54 in der Unterseite des Verbindungsstegs 35 ausgebildet.

Abweichend zur Fig. 5a ist bei dem Ausführungsbeispiel gemäß der Fig. 5b in der Oberseite des Verbindungsstegs 35 eine längliche Aussparung oder Nut 42 ausgebildet, die den gasdurchlässigen Abschnitt 54 weiter mit dem Innenraum der zugeordneten Aufnahme 34 verbindet, durch den das Gas zum Sterilisieren zusätzlich strömen kann, wodurch ein ausreichender Strömungsquerschnitt des Strömungskanals 65 auch bei einem vergleichsweise dünnen Klebemittelstreifen 49 sichergestellt werden kann. Selbstverständlich kann eine solche Aussparung oder Nut auch zusätzlich oder alternativ in der Unterseite der Schutzfolie 50 ausgebildet sein. Die Aussparung oder Nut 42 wird beim Verbinden von Schutzfolie 50 und Träger 31 mit verschlossen, beispielsweise durch ein Klebemittel, das in die Aussparung oder Nut 42 einfließt.

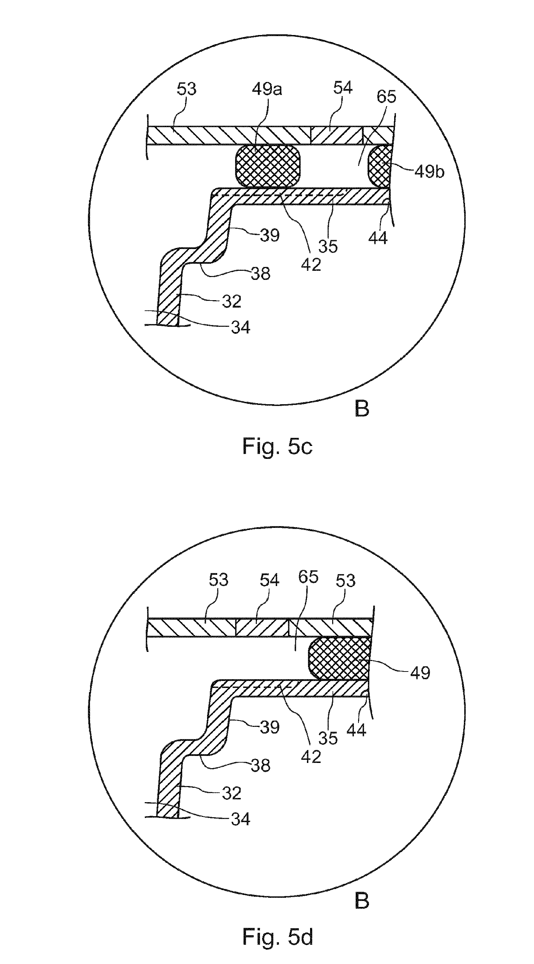

Abweichend zu den vorgenannten Ausführungsbeispielen ist gemäß der Fig. 5c zu beiden Seiten des gasdurchlässigen Abschnitts 54 ein Klebemittelstreifen 49a, 49b ausgebildet, sodass der gasdurchlässige Abschnitt 54 auf beiden Seiten gasdicht und steril gegen die Aufnahmen zu beiden Seiten des gasdurchlässigen Abschnitts 54 abgedichtet werden kann. Auch hier kann eine Aussparung oder Nut 42 zusätzlich vorgesehen sein, wie vorstehend beschrieben.

Abweichend zu den vorgenannten Ausführungsbeispielen ist gemäß der Fig. 5d der gasdurchlässige Abschnitt 54 innerhalb des umlaufend ausgebildeten Klebemittelstreifens 49 angeordnet, sodass ein vergleichsweise großer Strömungsquerschnitt für den Strömungskanal 65 bereitgestellt werden kann. Zum Verbinden von Schutzfolie 50 und Träger 31 ist auf eine ausreichende Größe des Klebemittelstreifens 49 und/oder ausreichende Druck- und/oder Wärmeeinwirkung zu achten, so dass der Klebemittelstreifen bis zum Rand der oberen Seitenwand 39 der Aufnahme fließt, um die Aufnahme vollständig gasdicht und steril gegen den gasdurchlässigen Abschnitt 54 abzudichten.

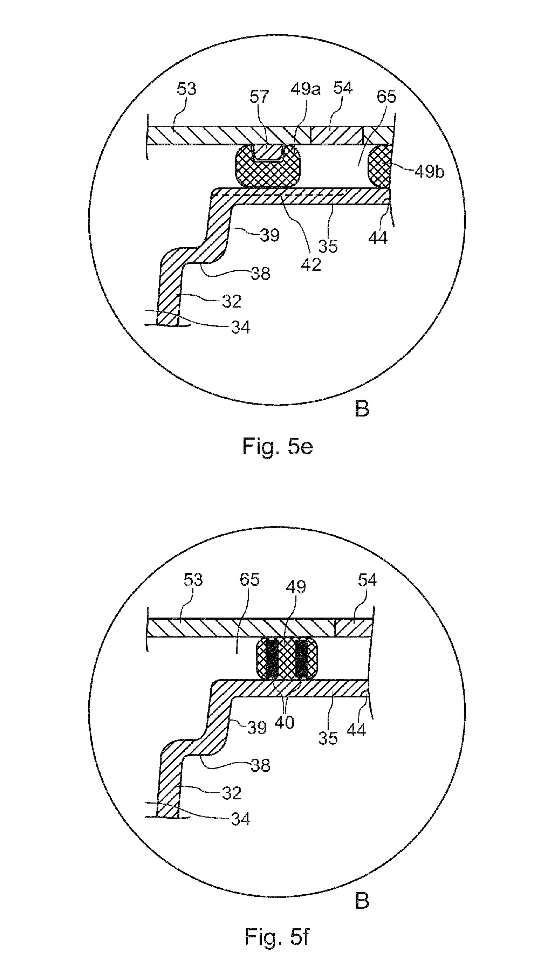

Abweichend zu den vorgenannten Ausführungsbeispielen ist gemäß der Fig. 5e auf der Unterseite des gasdurchlässigen Abschnitts 53 ein Verschlussstopfen 49a vorgesehen, der korrespondierend zum Querschnitt der Aussparung oder der Nut 42 ausgebildet ist. Durch Druckbeaufschlagung von oben während des Verbindens von Schutzfolie 50 und Träger 31 wird dieser Verschlussstopfen 49a in die zugeordnete Aussparung oder Nut 42 gedrückt, um diese gemeinsam mit dem einfließenden Klebemittel des Klebemittelstreifens 49a zu verschließen. Der Verschlussstopfen 49a kann an der Unterseite des gasundurchlässigen Abschnitts 53 als Kunststoffstopfen angespritzt oder befestigt sein oder kann einstückig mit dem gasundurchlässigen Abschnitt 53 ausgebildet sein. Sofern die Aussparung oder Nut 42 nicht in dem Verbindungssteg 35, sondern in der Unterseite der Schutzfolie 50 ausgebildet ist, ist der Verschlussstopfen 49a korrespondierend auf der Oberseite des Verbindungsstegs 35 ausgebildet.

Abweichend zu den vorgenannten Ausführungsbeispielen sind gemäß der Fig. 5f zusätzliche Abstandselemente 40 im Bereich des Strömungskanals 65 vorgesehen, um einen ausreichenden Strömungsquerschnitt sicherzustellen. Die Abstandselemente 40 können an die Unterseite der Schutzfolie 50 oder die Oberseite der Verbindungsstege 35 beispielsweise als Kunststoff angespritzt oder an diesen befestigt sein oder können einstückig mit diesen ausgebildet sein. Die Abstandselemente 40 können losbrechbar oder niederdrückbar ausgelegt sein, sodass diese bei Druckbeaufschlagung während des Verbindens von Schutzfolie 50 und Träger 31 abgebrochen oder gelöst werden, um in den Klebemittelrand eingebettet zu werden, der umlaufend um die jeweiligen Aufnahmen 34 herum ausgebildet wird.

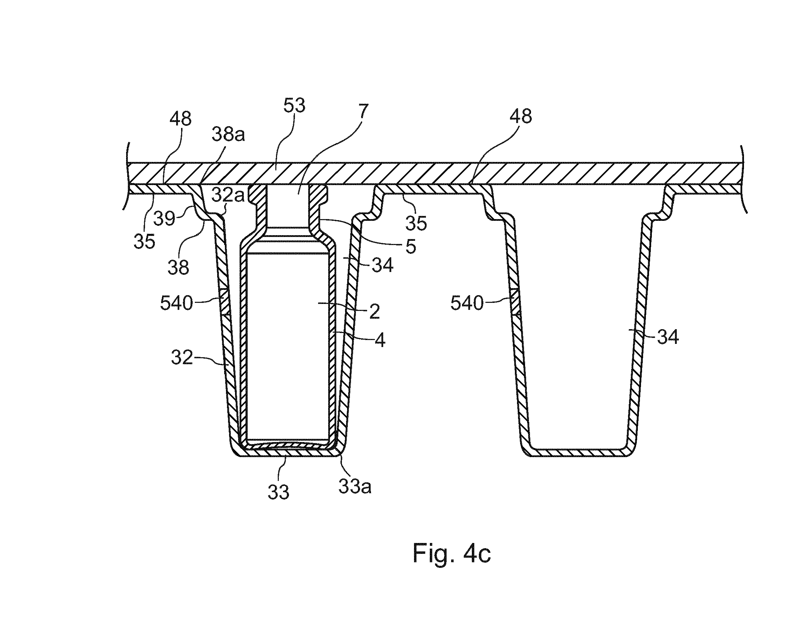

Während vorstehend beschrieben wurde, dass der selektiv gasdurchlässige Abschnitt in der Schutzfolie angeordnet ist, kann der selektiv gasdurchlässige Abschnitt gemäß weiteren Ausführungsformen auch unmittelbar in der Verpackungsstruktur ausgebildet sein. Dies ist beispielhaft in der Fig. 4c dargestellt, die in einem schematischen Teilschnitt eine Verpackungsstruktur gemäß einer weiteren Ausführungsform der vorliegenden Erfindung zeigt. Abweichend zur Ausführungsform gemäß der Fig. 4b ist gemäß der Fig. 4c der selektiv gasdurchlässige Abschnitt 540 unmittelbar in der zylindrischen Seitenwand 32 einer jeweiligen Aufnahme 34 ausgebildet. Dies lässt sich grundsätzlich durch Ausbilden einer Aussparung in der zylindrischen Seitenwand 32 und Schließen der Aussparung durch Verbinden einer selektiv gasdurchlässigen Folie 540 mit der Seitenwand 32 realisieren. Bei Ausführungsformen, bei denen der Träger der Verpackungsstruktur aus einem Verbundwerkstoff ausgebildet ist, lässt sich dies auch dadurch realisieren, dass die selektiv gasdurchlässige Folie 540 in eine Kunststoffeinlage eingearbeitet ist oder diese ausbildet, die Bestandteil des Verbundwerkstoffs ist. Alternativ kann der selektiv gasdurchlässige Abschnitt 540 auch in dem Boden 33 der jeweiligen Aufnahme 34 angeordnet sein. Alternativ kann der selektiv gasdurchlässige Abschnitt 540 auch ringförmig ausgebildet sein und sich um den gesamten Umfang der jeweiligen Aufnahme 34 erstrecken. In einem solchen Fall kann der selektiv gasdurchlässige Abschnitt 540 auch als separates ringförmiges Element zwischen der Schutzfolie 50 und der Verpackungsstruktur angeordnet werden. Bei den Ausführungsformen gemäß diesem Abschnitt braucht die Schutzfolie somit keinen gasdurchlässigen Abschnitt aufzuweisen. Sofern erforderlich, können die selektiv gasdurchlässigen Abschnitte nach dem Sterilisieren der Aufnahmen und/oder Behälter mittels gasundurchlässigen Abschnitten steril verschlossen werden, beispielsweise durch Aufkleben von gasundurchlässigen Streifen auf die jeweiligen gasdurchlässigen Abschnitte 540.

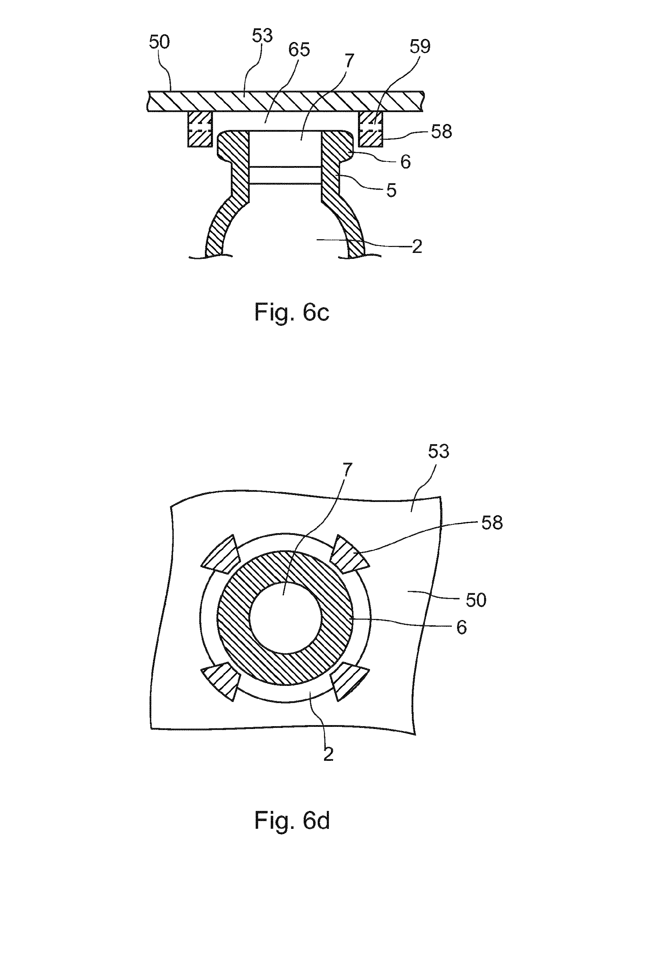

Anhand der Figuren 6c und 6d werden nachfolgend weitere Ausführungsformen der Schutzfolie beschrieben. Gemäß der Fig. 6c sind auf der Unterseite des gasundurchlässigen Abschnitts 53 der Schutzfolie Zentrierungs- und Positionierungsmittel 58 vorgesehen, die so mit dem oberen Rand der in den Aufnahmen des Trägers aufzunehmenden Behälter 2 zusammenwirken, dass diese in den Aufnahmen zentriert und geeignet positioniert sind. Um ein Einströmen des Gases in den Innenraum eines Behälters zu ermöglichen, können in diesen Zentrierungs- und Positionierungsmitteln 58 auch Strömungskanäle 59 ausgebildet sein. Derartige Zentrierungs- und Positionierungsmittel 58 können insbesondere auch als Abstandselemente eingesetzt werden, um einen ausreichenden Abstand zwischen dem oberen Rand des aufzunehmenden Behälters 2 und der Unterseite des gasundurchlässigen Abschnitts 53 zu gewährleisten. Zu diesem Zweck können die Zentrierungs- und Positionierungsmittel 58 insbesondere auch gestuft ausgebildet sein, mit einer Stufe (nicht gezeigt), die sich bis über den oberen Rand 6 des Behälters 2 hinaus radial einwärts erstreckt.

Die Fig. 6d zeigt eine weitere Ausführungsform solcher Zentrierungs- und Positionierungsmittel 58, die hier als Kreissegmente ausgebildet sind und unter bevorzugt gleichmäßigen Winkelabständen zueinander verteilt um den oberen Rand 6 des Behälters 2, angeordnet sind. Dabei kann das in die Aufnahmen einströmende Gas durch die Zwischenräume von benachbarten Zentrierungs- und Positionierungsmitteln 58 hindurch in den Innenraum des Behälters 2 einströmen. Die Zentrierungs- und Positionierungsmittel 58 können auch hier gestuft ausgebildet sein, mit einer Stufe (nicht gezeigt), die sich bis über den oberen Rand 6 des Behälters 2 hinaus radial einwärts erstreckt.

In dem Zustand gemäß der Fig. 1 oder 2 können die in den Aufnahmen der Verpackungsstruktur 30 aufgenommenen Behälter steril verpackt und mechanisch geschützt transportiert werden. Die Behälter können beispielsweise gefüllte und/oder ungefüllte Fläschchen (vials), Spritzen (syringe) und Doppelkammer-Spritzen (dual-chamber syringe) oder Karpulen (cartridges) und Doppelkammer-Karpulen (dual chamber cartridges) oder Vartridges sein. Diese können in der Verpackungsstruktur mit unterschiedlichen Größen (Länge und Durchmesser) sicher und ohne Glaskontakt transportiert werden. Die Seitenwände der Aufnahmen können durchsichtig ausgebildet sein, sodass die in den Aufnahmen aufgenommenen Behälter von außen sichtbar sind und visuell oder optoelektronisch beurteilt, geprüft oder untersucht werden können, beispielsweise mit Hilfe eines Laser- oder LED-Strahls oder einer optoelektronischen Erfassungseinrichtung. Auf diese Weise kann insbesondere auch eine Sichtverpackung (Blister) zur gleichzeitigen Aufnahme einer Mehrzahl von Behältern in Aufnahmen von Verpackungsuntereinheiten bereitgestellt werden, die einzeln und jeweils als steril abgedichtete Sichtverpackung abgetrennt werden können, wie vorstehend beschrieben. Die Behälter können stehend, liegend oder kopfüber aufbewahrt werden.

Die in der Verpackungsstruktur 30 steril aufbewahrten Behälter können selbstverständlich nach Abnehmen oder Öffnen der Schutzfolie von zumindest einer Aufnahme der Verpackungsstruktur 30 weiter verarbeitet oder bearbeitet werden. Diese Weiterverarbeitung oder Bearbeitung der Behälter kann erfolgen, während diese weiterhin in den Aufnahmen aufgenommen sind. Alternativ kann diese Weiterverarbeitung oder Bearbeitung der Behälter auch außerhalb der Aufnahmen erfolgen, beispielsweise in Prozessstationen, an denen die Behälter jeweils aus den Aufnahmen entnommen, weiter verarbeitet oder bearbeitet und abschließend wieder zurück in die Aufnahmen eingebracht werden. Durch Aufbringen der zuvor abgenommenen oder geöffneten Schutzfolie oder einer anderen (neuen) Schutzfolie kann die Verpackungsstruktur wieder verschlossen werden, bevorzugt steril, wie vorstehend beschrieben. Zu diesem Zweck kann die Verbindung von Schutzfolie und Träger mittels des gleichen Klebe- oder Versiegelungsmittels erfolgen, das zur Ausbildung der Verpackungsstruktur verwendet wurde. Oder es wird ein neues Klebe- oder Versiegelungsmittel zur Verbindung der Schutzfolie mit dem Träger geeignet aufgebracht. Zur erneuten Ausbildung der Verpackungsstruktur können die Aufnahmen des Trägers erneut in der vorstehend beschriebenen Weise durch Einströmen eines Gases durch die gasdurchlässigen Abschnitte der Schutzfolie sterilisiert werden.

Bei den Verfahrensschritten zur Bearbeitung der Verarbeitung der Behälter kann es sich insbesondere um einen der nachfolgenden Schritte handeln: Reinigung der Behälter, Wärmebehandlung der Behälter, Desinfektion der Behälter, Befüllen der Behälter mit einer Substanz für medizinische, pharmazeutische oder kosmetische Anwendungen, Gefriertrocknung der Substanz, Aufbringen einer Verschlusskappe auf einen Verschluss des Behälters, insbesondere einer Metallfolie durch Bördeln oder Crimpen, oder Aufbringen einer Kunststoffkappe und Verschließen des Behälters, Rütteln oder Schütteln des Behälters und/oder Wiegen des Behälters zwischen den einzelnen Verfahrensschritten.

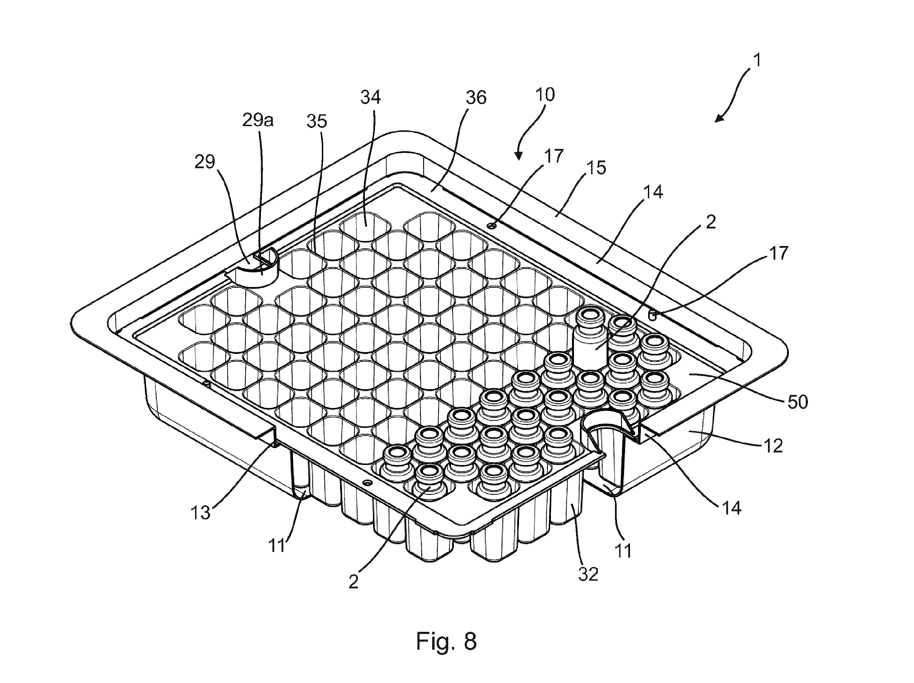

Eine Verpackungsstruktur 30, wie vorstehend beschrieben, kann in einem zusätzlichen Transport- und Verpackungsbehälter 10 transportiert werden, wie beispielhaft in der Fig. 8 gezeigt. Ein solcher Transport- und Verpackungsbehälter ist jedoch für einen sterilen Transport der Behälter nicht zwingend erforderlich.

Gemäß der Fig. 8 ist der Transport- und Verpackungsbehälter 10 im Wesentlichen kasten- oder wannenförmig ausgebildet und weist einen Boden 11, eine senkrecht von diesem abragende, umlaufend ausgebildete Seitenwand 12, eine von dieser im Wesentlichen rechtwinklig abragende Stufe 13, eine umlaufend ausgebildete obere Seitenwand 14 und einen oberen Rand 15 auf, der flanschartig ausgebildet ist. Die obere Seitenwand 14 kann unter einem geringen Neigungswinkel zur Senkrechten auf den Boden 11 geneigt ausgebildet sein, um das Einführen der Verpackungsstruktur 30 zu erleichtern. Ein derartiger Transport- und Verpackungsbehälter 10 ist bevorzugt aus einem Kunststoff ausgebildet, insbesondere durch Kunststoff-Spritzgusstechnik, und ist bevorzugt aus einem klaren, durchsichtigen Kunststoff ausgebildet, um eine optische Sichtkontrolle der in dem Transport- und Verpackungsbehälter 10 aufgenommenen Verpackungsstruktur 30 und der von dieser gehaltenen Behälter 2 zu ermöglichen.

Zur Aufnahme der Verpackungsstruktur 30 in dem Transport- und Verpackungsbehälter 10 kann diese von einem umlaufend ausgebildeten Randsteg umrandet sein. Ein solcher Randsteg kann auch entlang des Umfangsrands abschnittsweise durchgehend ausgebildet sein. Zur zuverlässigen Positionierung der Verpackungsstruktur 30 in dem Transport- und Verpackungsbehälter 10 weisen die Verpackungsstruktur 30 und der Transport- und Verpackungsbehälter 10 miteinander zusammenwirkende Positionierungsgebilde auf, die insbesondere formschlüssig zusammenwirken. So können an geeigneter Stelle, insbesondere auf der Stufe 13 oder auf Abstützflächen 18 des Transport- und Verpackungsbehälters 10 Positionierungsgebilde in Form von Vorsprüngen oder Aussparungen bzw. Vertiefungen ausgebildet sein, die mit korrespondierend ausgebildeten Aussparungen bzw. Vertiefungen oder Vorsprüngen der Verpackungsstruktur formschlüssig zusammenwirken, um die Verpackungsstruktur 30 präzise in dem Transport- und Verpackungsbehälter 10 zu positionieren. Zu diesem Zweck können insbesondere auf der Stufe 13 des Transport- und Verpackungsbehälters 10 mehrere zapfenartige Vorsprünge 17 ausgebildet sein, die in korrespondierend ausgebildete Zentrieröffnungen in einem Halterahmen der Verpackungsstruktur 30 zusammenwirken. Gemäß der Fig. 8 ist die Stufe 13 des Transport- und Verpackungsbehälters 10 als umlaufende, ebene Abstützfläche ausgebildet, auf welcher die Verpackungsstruktur 30 unmittelbar aufliegt. Gemäß weiteren Ausführungsformen können an den Seitenwänden 12 des Transport- und Verpackungsbehälters 10 auch weitere Abstützflächen 18 oder Abstützelemente ausgebildet sein, insbesondere in Form von Vorsprüngen. Auf diese Weise kann die Verpackungsstruktur 30 präzise in dem Transport- und Verpackungsbehälter 10 positioniert werden und die Mehrzahl von Fläschchen 2 auf diese Weise in einer regelmäßigen Anordnung und an präzise definierten Positionen in einem Transport- und Verpackungsbehälter 10 mit standardisierten Abmessungen angeordnet und gehalten werden.

Wenngleich in der Fig. 8 der Boden 11 des Transport- und Verpackungsbehälters 10 als geschlossen und einstückig mit der Seitenwand 12 ausgebildet dargestellt ist, kann das untere Ende des Transport- und Verpackungsbehälters 10 auch in der Art des oberen Endes geöffnet ausgebildet sein, insbesondere mit einem flanschartigen unteren Rand in der Art des oberen Rands 15 versehen sein.

Wie in der Fig. 8 dargestellt, ist in der regelmäßigen Anordnung gemäß der Fig. 8 die Mehrzahl von Fläschchen 2 in einer Matrixanordnung entlang von zwei zueinander orthogonalen Richtungen unter vorbestimmten konstanten Abständen zueinander verteilt angeordnet. Grundsätzlich sind auch weitere regelmäßige Anordnungen denkbar, beispielsweise können zueinander benachbarte Reihen bzw. Spalten von Aufnahmen auch um eine vorbestimmte Länge versetzt zueinander angeordnet sein, und zwar in einer wiederkehrenden Anordnung mit vorbestimmter Periodizität. Somit können automatisierte Fertigungsanlagen die Behälter 2 bei Übergabe an eine Bearbeitungsstation an präzise vorgebbaren Positionen erwarten, was den Automatisierungsaufwand erheblich verringert.

Damit die Verpackungsstruktur 30 leicht in den Transport- und Verpackungsbehälter 10 eingesetzt und aus diesem entnommen werden kann, sind an zwei Längsseiten der Verpackungsstruktur 30 Zugriffsöffnungen 29 ausgebildet, über welche Greifarme oder dergleichen die Verpackungsstruktur 30 greifen können. Die Zugriffsöffnungen 29 können, in Längs- oder Querrichtung der Verpackungsstruktur 30 betrachtet, versetzt zueinander angeordnet sein, was eine eineindeutige Positionierung der Verpackungsstruktur 30 in dem Transport- und Verpackungsbehälter 10 weiter erleichtert.

Das obere Ende des Transport- und Verpackungsbehälters 10, sowie soweit vorgesehen auch das untere Ende des Transport- und Verpackungsbehälters 10, kann mit einer Schutzfolie steril gegen die Umgebung abgeschlossen sein, insbesondere mit einem Geflecht aus Kunststofffasern, beispielsweise aus Polypropylen-Fasern (PP) oder einer Tyvek®-Schutzfolie, wie vorstehend im Zusammenhang mit der Verpackungsstruktur beschrieben.

Wie dem Fachmann beim Studium der vorliegenden Anmeldung ohne Weiteres ersichtlich sein wird, betrifft ein weiterer Gesichtspunkt der vorliegenden Erfindung die Verwendung einer Schutzfolie wie vorstehend beschrieben und beispielhaft in den Figuren 6a und 6b dargestellt, zur sterilen Verpackung einer Mehrzahl von Behältern für medizinische, pharmazeutische oder kosmetische Anwendungen in einer Verpackungsstruktur bzw. einem Verfahren, wie vorstehend beschrieben.

Wie dem Fachmann beim Studium der vorstehenden Beschreibung ohne Weiteres ersichtlich sein wird, können die einzelnen Gesichtspunkte und Merkmale der vorstehend beschriebenen Ausführungsbeispiele auch in beliebiger Weise miteinander kombiniert werden, was in zahlreichen weiteren Ausführungsformen und Modifikationen resultiert. Wie dem Fachmann beim Studium der vorliegenden Beschreibung ohne Weiteres ersichtlich sein wird, sollen sämtliche solche weiteren Ausführungsformen und Modifikationen von der vorliegenden Erfindung mit umfasst sein, solange diese nicht von dem allgemeinen Lösungsgedanken und dem Schutzbereich der vorliegenden Erfindung abweichen, wie in den beigefügten Patentansprüchen festgelegt.

## Patentansprüche

1. Verpackungsstruktur zur sterilen Verpackung mit einer Mehrzahl von Behältern (2) für medizinische, pharmazeutische oder kosmetische Anwendungen, die in der Verpackungsstruktur steril verpackt gegen die Umgebung aufgenommen sind, umfassend:
einen Träger (31), in welchem eine Mehrzahl von Aufnahmen (34) ausgebildet ist;
eine Mehrzahl von Behältern (2), die jeweils einzeln in den Aufnahmen aufgenommen sind; und
eine gasundurchlässige Schutzfolie (50), die mit der Oberseite des Träges (35) verbunden ist, um die Aufnahmen steril gegen die Umgebung zu versiegeln; wobei
die Aufnahmen jeweils von einem geschlossenen Boden (33) und einer umlaufenden Seitenwand (32) ausgebildet sind,
die dem jeweiligen Boden gegenüberliegenden oberen Enden der Aufnahmen offen ausgebildet sind,
an den oberen Enden der Aufnahmen umlaufend ausgebildete Verbindungsstege (35) vorgesehen sind, über welche benachbarte Aufnahmen miteinander verbunden sind,
die Schutzfolie entlang den Verbindungsstegen (35) mit diesen verbunden ist, sodass durch Abtrennung entlang den Verbindungsstegen (35) jeweils einzelne Verpackungs-Untereinheiten mit einer Aufnahme (34) abtrennbar sind, in denen jeweils ein einzelner Behälter (2) aufgenommen ist,
**dadurch gekennzeichnet, dass** den Aufnahmen (34) zumindest ein gasdurchlässiger Abschnitt (54; 540) zugeordnet ist, der als Abschnitt der gasundurchlässigen Schutzfolie (50) oder der Verpackungsstruktur (30) ausgebildet ist, sodass die Aufnahmen der Verpackungsstruktur (30) durch Einströmen eines Gases durch den zumindest einen gasdurchlässigen Abschnitt (54; 540) sterilisiert werden können.

2. Verpackungsstruktur nach Anspruch 1, wobei der zumindest eine gasdurchlässige Abschnitt (54) in der Schutzfolie ausgebildet ist, wobei sämtliche Aufnahmen (34) der Verpackungsstruktur durch Verbinden der Schutzfolie entlang den Verbindungsstegen jeweils einzeln steril und gegen den zumindest einen gasdurchlässigen Abschnitt abgedichtet verpackt sind.

3. Verpackungsstruktur nach Anspruch 1 oder 2, wobei jeder Aufnahme (34) ein gasdurchlässiger Abschnitt (54) zugeordnet ist, der nach dem Verbinden der Schutzfolie entlang den Verbindungsstegen nicht mit dem Innenraum der Aufnahme kommuniziert.

4. Verpackungsstruktur nach Anspruch 3, wobei sich vom Innenraum der jeweiligen Aufnahme (34) zu dem zugeordneten gasdurchlässigen Abschnitt (54) in der Oberseite des Trägers und/oder in der Unterseite der Schutzfolie eine Aussparung (42) erstreckt, die nach dem Verbinden der Schutzfolie entlang den Verbindungsstegen verstopft ist, sodass die Aufnahmen jeweils steril verpackt und gegen den jeweils zugeordneten gasdurchlässigen Abschnitt abgedichtet sind.

5. Verpackungsstruktur nach einem der Ansprüche 2 bis 4, wobei
der zumindest eine gasdurchlässige Abschnitt (54) von einer gasdurchlässigen Kunststofffolie ausgebildet ist, insbesondere einem Geflecht aus Kunststofffasern, oder von einem Abschnitt mit einer gitterartigen Struktur mit Durchbrechungen ausgebildet ist, und
wobei der zumindest eine gasundurchlässige Abschnitt (53) der Schutzfolie aus einer Kunststoff- oder Metallfolie oder einem Verbundwerkstoff daraus ausgebildet ist.

6. Verpackungsstruktur nach einem der Ansprüche 2 bis 5, wobei die Verpackungsstruktur aus einem Material ausgebildet ist, welches gegen Wasserstoffperoxid-Dampf (VHP) resistent ist.

7. Verfahren zur sterilen Verpackung einer Mehrzahl von Behältern (2) für medizinische, pharmazeutische oder kosmetische Anwendungen, mit den folgenden Schritten:
Bereitstellen eines Trägers (35), in welchem eine Mehrzahl von Aufnahmen (34) ausgebildet ist, wobei die Aufnahmen jeweils von einem geschlossenen Boden (33) und einer umlaufenden Seitenwand (32) ausgebildet sind, die dem jeweiligen Boden gegenüberliegenden oberen Enden der Aufnahmen offen ausgebildet sind und an den oberen Enden der Aufnahmen umlaufend ausgebildete Verbindungsstege (35) vorgesehen sind (S1);
Anordnen der Mehrzahl von Behältern (2) in den Aufnahmen (34) des Trägers (S2);
Bereitstellen einer gasundurchlässigen Schutzfolie (50);
Verbinden der Schutzfolie entlang den Verbindungsstegen (35) mit der Oberseite des Trägers (S5), um sämtliche Aufnahmen (34) mit den darin jeweils einzeln aufgenommenen Behältern (2) zu verpacken; und
Sterilisieren der Aufnahmen (34) mit den darin aufgenommenen Behältern und/oder der Innenräume der Behälter durch Einströmen eines Gases (S4) in die Aufnahmen (34) des Trägers und/oder in die Innenräume der Behälter durch zumindest einen gasdurchlässigen Abschnitt (54; 540), der als Abschnitt der gasundurchlässigen Schutzfolie (50) oder der Verpackungsstruktur (30) ausgebildet ist.

8. Verfahren nach Anspruch 7, bei dem die Schutzfolie den zumindest einen gasdurchlässigen Abschnitt (54) aufweist, mit den weiteren Schritten:
Anordnen der Schutzfolie (50) derart auf der Oberseite des Trägers, dass die Aufnahmen (34) des Trägers mit dem jeweils zugeordneten gasdurchlässigen Abschnitt (54) der Schutzfolie kommunizieren (S3); und
Sterilisieren der Aufnahmen (34) mit den darin aufgenommenen Behältern und/oder der Innenräume der Behälter durch Einströmen eines Gases durch den zumindest einen gasdurchlässigen Abschnitt (54; 540) in die Aufnahmen (34) des Trägers und/oder in die Innenräume der Behälter (S4); wobei
die Schutzfolie so entlang den Verbindungsstegen mit der Oberseite des Trägers verbunden wird, dass die Aufnahmen (34) der einzelnen Verpackungs-Untereinheiten jeweils einzeln steril und gegen den zumindest einen gasdurchlässigen Abschnitt abgedichtet verpackt sind.

9. Verfahren nach Anspruch 8, wobei die Aufnahmen (34) des Trägers mit dem jeweils zugeordneten gasdurchlässigen Abschnitt (54) über Strömungskanäle (65) kommunizieren, die zwischen der Oberseite des Trägers (31) und der Schutzfolie (50) verlaufen und/oder die in der Oberseite des Trägers und/oder der Unterseite der Schutzfolie ausgebildet sind und die beim Verbinden der Schutzfolie entlang den Verbindungsstegen verschlossen werden.

10. Verfahren nach Anspruch 8 oder 9, wobei der zumindest eine gasdurchlässige Abschnitt (54) außerhalb eines jeweiligen Verbindungsbereichs (48) angeordnet ist, der eine jeweilige Aufnahme entlang den zugeordneten Verbindungsstegen (35) umlaufend umgibt, wobei das Verbinden der Schutzfolie entlang den Verbindungsstegen mittels Heißverklebung oder Heißversiegelung erfolgt, wobei vor dem Anordnen der Schutzfolie auf der Oberseite des Trägers ein Heißklebe- oder Heißversiegelungsrand (49; 49a) auf der Unterseite der Schutzfolie, die der Oberseite des Trägers zugewandt ist, und/oder auf der Oberseite des Trägers ausgebildet wird, der zumindest im Bereich des zugeordneten Strömungskanals (65) unterbrochen ist und durch die Heißverklebung oder Heißversiegelung verschlossen wird.

11. Verfahren nach Anspruch 10, wobei auf der dem Heißklebe- oder Heißversiegelungsrand (49a) abgewandten Seite des gasdurchlässigen Abschnitts (54) ein weiterer Heißklebe- oder Heißversiegelungsabschnitt (49b) vorgesehen ist.

12. Verfahren nach einem der Ansprüche 9 bis 11, wobei die Höhe der Strömungskanäle (65) mittels Abstandselementen (40, 41) festgelegt wird, die auf der Unterseite der Schutzfolie und/oder der Oberseite des Trägers vorgesehen sind, wobei zumindest einige der Abstandselemente (40) beim Verbinden der Schutzfolie mit der Oberseite des Trägers niedergedrückt oder losgebrochen werden, bevorzugt innerhalb eines Heißklebe- oder Heißversiegelungsrands (49; 49a).

13. Verfahren nach einem der Ansprüche 7 bis 12, wobei auf der Unterseite der Schutzfolie (50) eine Mehrzahl von Zentrierungs- oder Positionierungsmitteln (58) vorgesehen ist, um die Behälter jeweils in einer Aufnahme des Trägers zu zentrieren oder zu positionieren, insbesondere um einen vorbestimmten Abstand zwischen der Unterseite der Schutzfolie und einem oberen Rand der Behälter festzulegen, wobei die Zentrierungs- oder Positionierungsmittel (58) bevorzugt einen Strömungskanal (58) aufweisen oder ausbilden, durch den das Gas in die Aufnahmen (34) des Trägers und/oder in die Innenräume der Behälter zum Sterilisieren einströmt.

14. Verfahren zur Bearbeitung oder Verarbeitung von Behältern (2) für medizinische, pharmazeutische oder kosmetische Anwendungen unter Verwendung einer Verpackungsstruktur (30) nach einem der Ansprüche 1 bis 6 oder einer Verpackungsstruktur (30), die nach einem Verfahren nach einem der Ansprüche 7 bis 13 hergestellt ist, mit den folgenden Schritten:
Bereitstellen der Verpackungsstruktur (30) mit einer Mehrzahl von Aufnahmen, in denen jeweils ein Behälter (2) steril verpackt aufgenommen ist;
Abnehmen oder Öffnen der Schutzfolie (50) von zumindest einer Aufnahme (34) der Verpackungsstruktur;
Bearbeitung oder Verarbeitung des jeweiligen Behälters der zumindest einen Aufnahme (34); und
Aufbringen der zuvor abgenommenen oder geöffneten Schutzfolie (50) oder einer anderen Schutzfolie, um die zumindest eine Aufnahme zu verschließen, bevorzugt steril zu verschließen, wobei die jeweiligen Behälter zur Bearbeitung oder Verarbeitung aus den Aufnahmen entnommen werden und nach der Bearbeitung oder Verarbeitung wieder in die Aufnahmen eingesetzt werden.

15. Verwendung einer gasundurchlässigen Schutzfolie (50) mit zumindest einem gasdurchlässigen Abschnitt (54) zur Herstellung einer Verpackungsstruktur nach einem der Ansprüche 1 bis 6 oder in einem Verfahren zur sterilen Verpackung einer Mehrzahl von Behältern nach einem der Ansprüche 7 bis 13 oder in einem Verfahren zur Bearbeitung oder Verarbeitung von Behältern nach Anspruch 14, wobei
der zumindest eine gasdurchlässige Abschnitt (54) der Schutzfolie von einer gasdurchlässigen Kunststofffolie ausgebildet ist, insbesondere einem Geflecht aus Kunststofffasern, oder von einem Abschnitt mit einer gitterartigen Struktur mit Durchbrechungen ausgebildet ist, und
wobei der zumindest eine gasundurchlässige Abschnitt (53) der Schutzfolie aus einer Kunststoff- oder Metallfolie oder einem Verbundwerkstoff daraus ausgebildet ist.
